# EUROPEAN PATENT SPECIFICATION

(11) **EP 3 214 609 B1**
(45) Date of publication and mention of the grant of the patent: **05.01.2022**
(21) Application number: 15855515.1
(22) Date of filing: 30.10.2015
(51) Int. Cl.: G08C 19/00, A61B 5/00, G08C 17/00

(54) **SENSOR DEVICE, SENSOR SYSTEM**
SENSORVORRICHTUNG, SENSORSYSTEM
DISPOSITIF DE CAPTEUR, SYSTÈME DE CAPTEUR

(30) Priority: 30.10.2014 JP 2014221281
(43) Date of publication of application: 06.09.2017
(73) Proprietor: FUJIFILM Corporation, Tokyo 106-8620 (JP)
(72) Inventor: SAWANO, Mitsuru, Shizuoka 421-0396 (JP)
(74) Representative: Hoffmann Eitle
(86) International application number: PCT/JP2015/080761
(87) International publication number: WO 2016/068295

(56) References cited:
- EP-A2- 1 587 141
- WO-A1-2004/046704
- JP-A- 2005 340 479
- JP-A- 2008 527 531
- JP-A- 2011 050 645
- JP-A- 2014 131 426
- US-A1- 2002 137 998
- US-A1- 2004 000 713
- US-A1- 2009 149 722
- US-A1- 2011 188 210
- US-A1- 2012 101 540
- US-A1- 2013 211 213

## Description

### BACKGROUND OF THE INVENTION

### 1. Field of the Invention

The present invention relates to a sensor device and a sensor system. More specifically, the present invention relates to a sensor device and a sensor system for a living body that are attached to a tooth or the like.

### 2. Description of the Related Art

A sensor device that wirelessly transmits a sensor signal to the outside has been proposed. The use of such a sensor device as, for example, a biosensor has been studied.

JP2011-512049A discloses a bidirectional communication device including a mouth-mounted communication device, a body sensor, and a coupling unit that is coupled to the mouth-mounted communication device and the body sensor and that is adapted to communicate with a remote station.

JP2013-539692A discloses a sensor system that is implanted into a living body and that includes: a measurement unit that measures a parameter of the living body to generate a corresponding measured signal; a transmission unit that transmits a signal using the measured signal; a control and the evaluation unit that controls transmission of the transmission signal of the transmission unit by processing the measured signal; an energy storage unit that supplies energy to each unit; and a casing that at least partially surrounds the measurement unit, the transmission unit, the control and evaluation unit, and the energy storage unit.

JP2014-160258A discloses a contact lens that can guide light to the pupil and that includes a semiconductor element including: a functional layer that functions according to control information; a control layer that controls the functional layer according to control information; a communication layer that can communicate with an external device; and a power supply layer for supplying electric power to each layer.

Another example can be seen in documents US 2011/188210 A1, US 2013/211213 A1 and US 2012/101540 A1.

### SUMMARY OF THE INVENTION

In the sensor device, low power consumption and a smaller size have been demanded in recent years. In biological sensor devices, the above characteristics are more required.

In JP2011-512049A, however, since the sensor signal is directly transmitted to the external device, the amount of data communication is large and the power consumption is large. In addition, although it will be described in JP2011-512049A that a replaceable or permanent battery can be included in an assembly as a power supply, inclusion of these batteries in the assembly tends to increase the size of the assembly.

In JP2013-539692A, a battery, such as a lithium ion battery or a film battery, is used as the energy storage unit. Accordingly, the sensor device disclosed in JP2013-539692A tends to be large. In addition, since these batteries (energy storage units) can not be manufactured by a semiconductor process, it is necessary to manufacture the batteries by a process different from the process for the measurement unit and the like. Accordingly, poor productivity has been a problem.

In the semiconductor element disclosed in JP2014-160258A, since data obtained from the functional layer is recorded in a read only memory (ROM) and is transmitted to the external device, the amount of data communication is large and the power consumption is large.

Therefore, it is an object of the present invention to provide a sensor device and a sensor system that consume less power and can be miniaturized.

As a result of intensive studies, the present inventors have found that the above object can be achieved by the following configuration, and have completed the present invention. According to the invention, a sensor device according to claim 1 is provided. The dependent claims define various embodiments of the invention.

### BRIEF DESCRIPTION OF THE DRAWINGS

Fig. 1 is a schematic diagram of a sensor device of a first example which does not form part of the present invention.
Fig. 2 is a perspective view of a unit of the sensor device.
Fig. 3 is an exploded perspective view of the unit shown in Fig. 2.
Fig. 4 is a flowchart showing an example of the algorithm of pass or fail determination in a logic portion.
Fig. 5 is a flowchart showing an example of the algorithm of rank determination in a logic portion.
Fig. 6 is a flowchart showing an example of the algorithm for determining the replacement timing of a sensor device.
Fig. 7 is a schematic plan view of a unit in a sensor device of a second example which does not form part of the present invention.
Fig. 8 is an exploded perspective view of a unit in a sensor device of an embodiment of the present invention.
Fig. 9 is a schematic plan view of a unit in a sensor device of a third example which does not form part of the present invention.
Fig. 10 is an exploded perspective view of a unit in a sensor device of a fourth example which does not form part of the present invention.

### DESCRIPTION OF THE PREFERRED EMBODIMENTS

The description of constituent elements in the present invention described below may be made based on representative embodiments of the present invention, but the present invention is not limited to such embodiments and it is defined by appended claims 1-10.

In this specification, the numerical range expressed using "to" means a range including numerical values described before and after "to" as a lower limit and an upper limit.

In this specification, the term "step" includes not only an independent step but also a case where the intended effect of the step can be achieved even in a case where the step cannot be clearly distinguished from other steps.

A sensor device of the present invention has: a unit having at least one type of power supply portion selected from a power generation portion and a wireless power supply portion, a sensor portion that detects target information and outputs a signal, a logic portion that performs determination (pass or fail determination, rank determination, or the like) based on the signal output from the sensor portion, and a wireless communication portion for transmitting the determination result to the outside; and a sealing material that covers at least a part of the outer periphery of the unit. The respective portions are formed on a plurality of semiconductor substrates, the semiconductor substrates on which the respective portions are formed are laminated, and these portions are electrically connected (wired or wirelessly connected) to each other.

In a unit of a conventional sensor device, electronic components having functions of respective portions, such as sensor elements, are mounted on a circuit board. However, according to the present invention, the unit has respective portions formed on a plurality of semiconductor substrates, the semiconductor substrates on which the respective portions are formed are laminated, and these portions are electrically connected to each other. Accordingly, since a circuit board (so-called printed circuit board) is not used, the size of the sensor device can be reduced.

In addition, since the logic portion performs pass or fail determination, rank determination, or the like based on the signal output from the sensor portion and the wireless communication portion transmits the determination result to the outside, it is possible to reduce the amount of data communication with respect to the external device. As a result, it is possible to reduce the power consumption of the sensor device.

In addition, since the sensor device of the present invention has at least one type of power supply portion selected from the power generation portion and the wireless power supply portion, it is possible to save time and effort required for battery replacement or the like. Therefore, since the sensor device can be kept attached to an attachment target for a long period of time, the sensor device can be suitably used as a biological sensor device. In particular, the sensor device is suitable as a sensor device that is attached to a tooth.

Hereinafter, the sensor device of the present invention will be described.

### <First example>

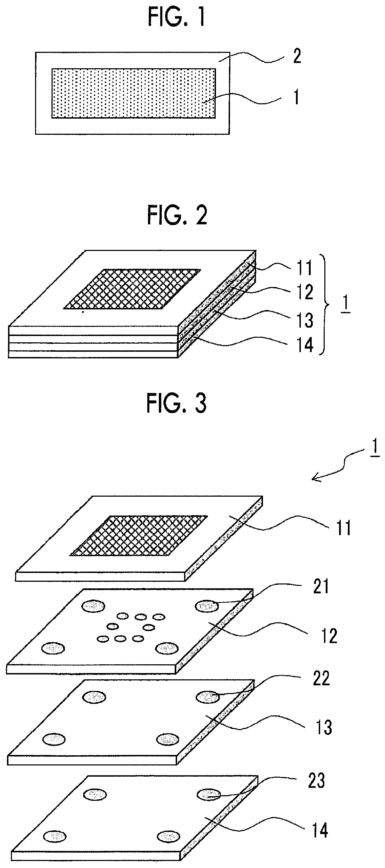

A first example which is not part of the sensor device of the present invention will be described with reference to Figs. 1 to 3.

Fig. 1 is a schematic diagram of the sensor device, Fig. 2 is a perspective view of a unit 1, and Fig. 3 is an exploded perspective view of the unit 1.

As shown in Fig. 1, the sensor device has the unit 1 and a sealing material 2 that covers at least a part of the outer periphery of the unit 1.

The shape of the sensor device is not particularly limited, and various shapes, such as a rectangular shape, a sheet shape, a spherical shape, and an ellipsoid shape, can be adopted.

In the sensor device of the present example, the maximum outer diameter is preferably 10 mm or less, more preferably 3 mm or less. In the present example, the maximum outer diameter means a diagonal line in a case where the sensor device has a rectangular shape or a sheet shape. In a case where the sensor device has a spherical shape, the maximum outer diameter means the diameter of the sensor device. In a case where the sensor device is an ellipsoid, the maximum outer diameter means the longest one of diameter axes of the ellipsoid. The lower limit is not particularly limited, but is preferably, for example, 0.1 mm or more from the viewpoint of handleability and the like.

In a case where the sensor device of the present example has a sheet shape, the thickness is preferably 0.001 mm to 3 mm. The lower limit is more preferably 0.005 mm or more, and even more preferably 0.01 mm or more. The upper limit is more preferably 2 mm or less, and even more preferably 1 mm or less. The length of the longitudinal side of the vertical and horizontal sides is preferably 1 mm to 20 mm. The upper limit is more preferably 10 mm or less, and even more preferably 5 mm or less.

In a case where the sensor device of the present example has a rectangular shape, the thickness is preferably 0.01 mm to 10 mm. The lower limit is more preferably 0.05 mm or more, and even more preferably 0.1 mm or more. The upper limit is more preferably 5 mm or less, and even more preferably 3 mm or less. The length of the longitudinal side of the vertical and horizontal sides is preferably 0.01 mm to 10 mm. The upper limit is more preferably 5 mm or less, and even more preferably 3 mm or less.

In a case where the sensor device of the present example has a spherical shape, the diameter is preferably 0.001 mm to 10 mm. The lower limit is more preferably 0.05 mm or more, and even more preferably 0.1 mm or more. The upper limit is more preferably 5 mm or less, and even more preferably 3 mm or less.

In a case where the sensor device of the present example is an ellipsoid, the length of the longest diameter axis is preferably 0.001 mm to 10 mm. The lower limit is more preferably 0.05 mm or more, and even more preferably 0.1 mm or more. The upper limit is more preferably 5 mm or less, and even more preferably 3 mm or less.

The sealing material 2 is preferably a material that is non-biodegradable and innocuous to a living body. In a case where the sensor device is attached to a tooth, a white material is preferable. The term "non-biodegradable" means not to be decomposed by the action of microorganisms.

Examples of the material that is non-biodegradable and innocuous to a living body include silicone resin, fluororesin, polyamide resin, polyimide resin, ethylene-vinyl acetate copolymer resin, paraxylylene resin, and the like.

As commercially available products, TEFLON (registered trademark, manufactured by Du Pont), PARYLENE (registered trademark, manufactured by Japan Parylene Co., Ltd.), and the like can be mentioned.

A thinnest part of the sealing material 2 is preferably 0.1 µm or more, more preferably 0.2 µm or more, and even more preferably 1 µm or more. If the film thickness is within the above range, the protection function of the unit 1 can be sufficiently obtained. From the viewpoint of miniaturization of the sensor device, the upper limit of the thinnest part of the sealing material 2 is preferably 200 µm or less, more preferably 100 µm or less, and even more preferably 50 µm or less.

Although the entire outer periphery of the unit 1 is covered with the sealing material 2 in Fig. 1, a part of the outer periphery of the unit 1 may be exposed from the sealing material.

In a case where the sensor device is used for a living body, it is preferable that the entire outer periphery of the unit 1 is covered with the sealing material 2 from the viewpoint of safety with respect to the living body.

As shown in Figs. 2 and 3, in the unit 1, a semiconductor substrate 14 having a wireless communication portion, a semiconductor substrate 13 having at least one type of power supply portion selected from a power generation portion and a wireless power supply portion, a semiconductor substrate 12 having a logic portion, and a semiconductor substrate 11 having a sensor portion are laminated in this order.

It is preferable that the semiconductor substrates are bonded to each other with an adhesive (not shown). Examples of the adhesive include epoxy resin, polyimide resin, acrylic resin, and the like. Underfill agents described in JP2012-025847A, JP2010-132793A, and JP2009-032732A can also be used.

The respective portions formed on the semiconductor substrates are electrically connected to each other through penetrating electrodes 21 to 23. The penetrating electrodes are connected to each other by solder bumps (not shown), copper bumps (surfaces may be plated with nickel, tin, gold, or the like), paste of metal (silver, copper, or the like), nanoparticles of metal (silver, copper, or the like), nanopillars of metal (silver, copper, aluminum, or the like), or the like.

In the present example, as shown in Figs. 2 and 3, in the unit 1, the semiconductor substrate 11 having a sensor portion and the semiconductor substrate 14 having a wireless communication portion are disposed at the outermost layers.

Since the semiconductor substrate 11 having a sensor portion is disposed at the outermost layer, the sensitivity as a sensor is good. In addition, since the semiconductor substrate 14 having a wireless communication portion is disposed at the outermost layer, communication with the outside is good.

In Figs. 2 and 3, the same effect can be obtained even if the lamination order of the semiconductor substrate 12 having a logic portion and the semiconductor substrate 13 having a power supply portion is changed.

In the present example, as shown in Fig. 3, the respective portions on semiconductor substrates are electrically connected to each other using the penetrating electrodes 21 to 23. However, the respective portions may be electrically connected to each other using a technique, such as wire bonding and wireless electromagnetic coupling, instead of the penetrating electrodes. Lamination based on wireless electromagnetic coupling is a technique of electrically connecting respective portions to each other by arranging coils on respective semiconductor substrates so that the coils face each other between the upper and lower semiconductor substrates. Regarding the laminating based on wireless electromagnetic coupling, for example, JP2011-086738A and JP2012-209769A can be preferred to.

In the present invention, known semiconductor substrates can be used without limitation. For example, a silicon substrate, a SiC substrate, a SiGe substrate, a ZnS substrate, a ZnSe substrate, a GaAs substrate, an InGaAs substrate, an InP substrate, a GaN substrate, and the like can be mentioned. In the present invention, each semiconductor substrate is a substrate for forming each function, such as a sensor portion, on the surface to form elements, and is different from a circuit board (a printed circuit board, a printed wiring board, and a substrate) on which electronic components (semiconductor elements and the like) as chips are to be mounted.

Hereinafter, each portion of the unit 1 will be described.

### «Sensor portion»

In the sensor device of the present invention, the sensor portion detects target information and outputs a signal, and can be selected according to the application. For example, an image sensor, a visible light sensor, an infrared light sensor, an ultraviolet light sensor, a temperature sensor, a humidity sensor, a pressure sensor, a tactile sensor, an acceleration sensor, a gyro sensor (magnetic sensor), a microphone (sound sensor), a gas sensor, an ion sensor, a biosensor, an electric resistance sensor, an electric impedance sensor, a proximity sensor, an electromagnetic wave sensor, a radiation sensor, a global positioning system (GPS) receiving sensor, and the like can be mentioned.

As the image sensor, a solid-state imaging device, such as a charge coupled device (CCD) and a complementary metal oxide semiconductor (CMOS), can be mentioned. The image sensor can be applied as an image sensor of visible light, infrared light, and ultraviolet light, and the like according to a semiconductor or an optical filter to be used.

The pressure sensor, the tactile sensor, the acceleration sensor, the gyro sensor, the microphone, and the like can also be manufactured by applying the semiconductor processing technology of the MEMS, and these are sometimes collectively referred to as a MEMS sensor.

Even in a case where the sensor device is attached to a tooth, all of the sensors described above can be applied, but it is preferable to use the image sensors and various optical sensor portions in combination with a light emitting device (energy generation portion). The reason is as follows. In the oral cavity, external light is not stably emitted. Accordingly, it is possible to increase the detection accuracy by using active sensing (emitting pulse light from the light emitting device for a short time and detecting reflected light or transmitted light thereof in synchronization with the light emission pulse) rather than passive sensing (detecting light from the outside in a passive manner in a state in which there is no light emitting device). As a result, it is possible to reduce power consumption for achieving the same accuracy.

### «Logic portion»

In the sensor device of the present invention, the logic portion performs determination (for example, pass or fail determination and rank determination) based on the signal output from the sensor portion. For example, a logic portion having a memory circuit (temporary memory) for recording the output signal of the sensor portion, a memory circuit (long-term memory) for recording a reference value for determination (determination reference value), and an arithmetic circuit for performing determination can be mentioned. The temporary memory can also be omitted.

Since the sensor device of the present invention has a logic portion that performs the above determination, it is possible to transmit only the determination result (for example, 2 bits) compared with a case of transmitting all pieces of digitized data of the sensor output (for example, 16 bits). Therefore, since the amount of data communication in the wireless communication portion is reduced, it is possible to reduce the power consumption.

In the determination of the logic portion, by reducing the number of ranks, it is possible to reduce the load on the logic portion. Therefore, it is possible to further reduce the power consumption. The number of ranks is preferably 256 or less, more preferably 64 or less, and further preferably 16 or less.

In the memory circuit (long-term memory) of the logic portion, it is preferable to record identification information unique to the sensor device in addition to the reference value for determination. As the identification information unique to the sensor device, for example, code information indicating the type, manufacturer, serial number, and the like of the sensor can be mentioned.

In the sensor device of the present invention, the logic portion may further have an analog-to-digital conversion portion (analog-to-digital conversion circuit) for digitally converting the signal output from the sensor portion. By digitally converting the signal output from the sensor portion, the determination circuit of the logic portion can be formed by only a digital circuit. Accordingly, in the case of complicated multilevel rank determination in which a plurality of determination reference values are set or in the case of determination based on a combination of a plurality of sensor signals (for example, rank A in a case where the temperature is 37°C or higher and the reflectance of visible light is 10% or less, rank B in a case where the temperature is 37°C or higher and the reflectance exceeds 10%, and rank C in a case where the temperature is lower than 37°C regardless of the reflectance), the circuit size can be reduced. As a result, it is possible to reduce the power consumption. On the other hand, in the case of simple determination (for example, pass if the sensor output is 1 V or higher and fail if the sensor output is lower than 1 V), a method of directly determining an analog signal by an analog circuit (transistor or the like) without analog-to-digital conversion is advantageous in that the total circuit size can be reduced by reducing the size of the logic circuit.

The analog-to-digital conversion portion can also be formed on a semiconductor substrate different from the logic portion.

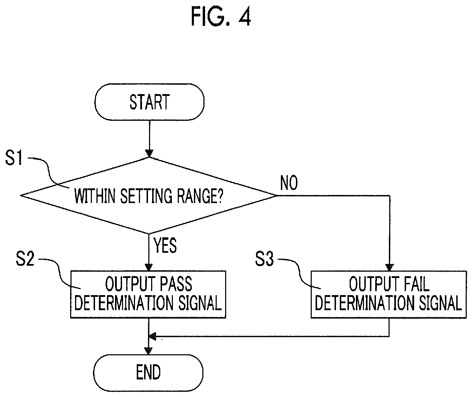

An example of the algorithm of pass or fail determination in the logic portion will be described with reference to Fig. 4.

As shown in Fig. 4, when a signal from the sensor portion is input to the logic portion, it is determined whether or not the input signal is in a setting range (step S1). If the input signal is in the setting range, a pass determination signal is output (step S2), and the process ends. If the input signal is outside the setting range, a fail determination signal is output (step S3), and the process ends.

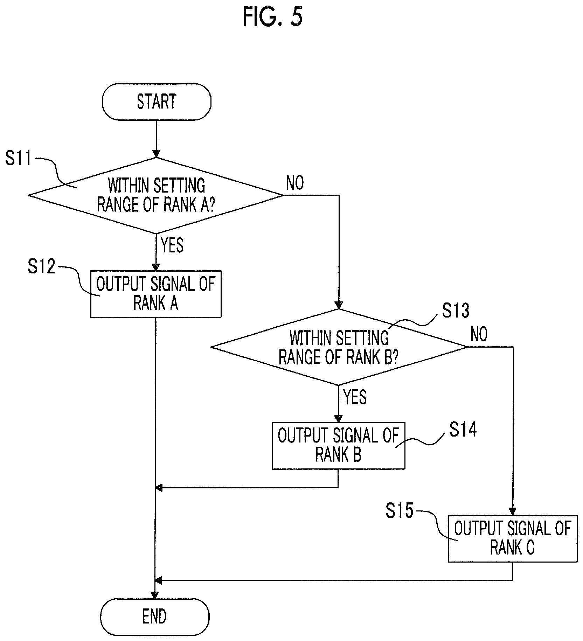

An example of the algorithm of rank determination in the logic portion will be described with reference to Fig. 5.

When a signal from the sensor portion is input to the logic portion, it is determined whether or not the input signal is in the setting range of the rank A (step S11). If the input signal is in the setting range, a signal of the rank A is output (step S12), and the process ends. If the input signal is outside the setting range, it is determined whether or not the input signal is in the setting range of the rank B (step S13). If the input signal is in the setting range, a signal of the rank B is output (step S14), and the process ends. If the input signal is outside the setting range of the rank B, a signal of the rank C is output (step S15), and the process ends.

In the sensor device of the present invention, it is preferable that the memory circuit (long-term memory) of the logic portion records at least one selected from the operation start date and time of the sensor device, the operating time of the sensor device, and operation examination information of each portion of the unit. In this aspect, it is preferable that the arithmetic circuit of the logic portion is configured to detect at least one piece of warning information, which is selected from the replacement timing of the sensor device and an abnormality in operation examination information, based on the recorded information.

The logic portion configured as described above has a function as an operation state management portion that manages the operation state of the sensor device.

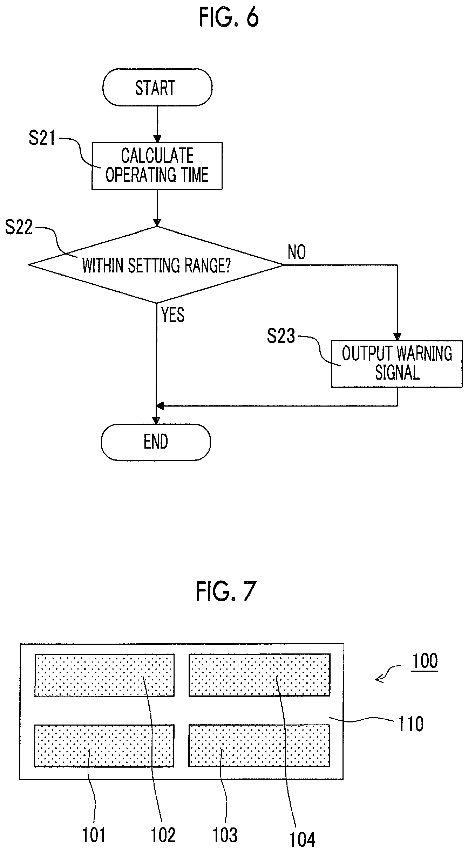

Determination of the replacement timing of the sensor device can be performed as follows, for example. As shown in Fig. 6, the operating time of the sensor device is calculated by comparing the operation start date and time of the sensor device recorded in the memory circuit with the current date and time (step S21). Then, it is determined whether or not the operating time of the sensor device is in a setting range (step S22). If the operating time of the sensor device is in the setting range, the process ends. On the other hand, in a case where the operating time of the sensor device is out of the setting range, it is determined that the lifespan has ended, and a warning signal for notification of the replacement timing of the sensor device is output. In a case where the operating time of the sensor device is recorded in the memory circuit, step S21 can be omitted.

Examples of the abnormality in operation examination information include an abnormality in the sensor, an abnormality in the logic circuit, an abnormality in the power supply circuit, an abnormality in the communication circuit, and the like. However, it is not necessary to analyze an abnormality in individual circuit portions and it is only necessary to determine whether or not there is any abnormality somewhere in the device. Accordingly, it is an object to detect whether there is any abnormality somewhere in the device. This is because, even if there is an abnormality in a part, it is not possible to repair only the part and the only final method is to replace the entire device.

The abnormality in operation examination information can be detected as follows, for example.

A method of detecting an abnormality in operation examination information by forming a circuit for disconnection check in a sensor device, transmitting a confirmation signal to the circuit for disconnection check, and determining that an abnormality, such as disconnection, has occurred in a case where the value is out of a predetermined range compared with an output value recorded in the memory circuit in advance can be mentioned.

In the sensor device, in a case where a light emitting diode (LED) is made to emit light and the amount of light is measured by the sensor portion, it is possible to use a method in which the LED is pulse-emitted at a prescribed examination value, a sensor reception signal is monitored, and it is determined that an abnormality has occurred in a case where the value of the signal deviates from the acceptance range that is set in advance and is recorded in the memory circuit, thereby detecting the abnormality in operation examination information.

The algorithm for determining the replacement timing of the sensor device and the algorithm for detecting an abnormality in operation examination information can also be performed by an external device. For example, it is possible to store identification information unique to the sensor device, the operation start date and time of the sensor device, and the algorithm for detecting an abnormality in operation examination information in association with each other and to perform the above-described determination in the external device.

It is preferable that the sensor device of the present invention is configured such that, in a case where an abnormality in operation examination information is detected, the logic portion corrects one or more selected from the signal output from the sensor portion and the determination reference value in the logic portion and performs determination, such as the pass or fail determination or the rank determination described above. That is, in a case where an abnormality in operation examination information is detected, it is preferable that the logic portion is configured to perform determination using one of the following methods.
(1) A measured value of the signal output from the sensor portion is corrected according to the abnormality in operation examination information, and the logic portion performs the above-described determination based on the sensor output after the correction.
(2) A determination reference value recorded in the memory circuit of the logic portion is corrected according to the abnormality in operation examination information, and the logic portion performs the above-described determination based on the determination reference value after the correction.
(3) A measured value of the signal output from the sensor portion and a determination reference value recorded in the memory circuit of the logic portion are corrected according to the abnormality in operation examination information, and the logic portion performs the above-described determination based on the sensor output and the determination reference value after the correction.

According to this aspect, it is possible to accurately perform detection in the sensor portion while suppressing the frequency of replacing the sensor device. By suppressing the frequency of replacing a sensor device, the sensor device of the present invention can be preferably used for applications in which it is difficult to frequently replace a sensor device, such as a biological sensor device. Among the above, the aspect (2) is particularly preferable. According to this aspect, it is sufficient to rewrite the determination reference value recorded in the memory circuit. Therefore, since it is possible to reduce the load on the logic circuit, a further reduction in power consumption can be expected. For example, by rewriting the "determination reference value under the normal conditions" to "determination reference value under the normal conditions × Z", the logic portion can output the same determination result as the determination result in the normal case.

Correction of the output of the sensor portion can be performed as follows, for example.

For example, in the sensor device, in a case where a light emitting diode (LED) is made to emit light and the amount of light is measured by the sensor portion, a standard reflection plate (high reflectance) is placed in front of the sensor portion, the LED is made to emit light and the amount of reflected light is monitored, and the output signal of the sensor portion is compared with the normal value recorded in the memory circuit. Then, a standard reflection plate (low reflectance) is placed and the same operation is performed. A correction value is calculated from the two pieces of data and the calculated correction value is recorded in the memory circuit, and the measured value of the signal output from the sensor portion is corrected with the correction value. The above-described pass or fail determination, rank determination, and the like are performed using a value obtained by correcting the measured value of the sensor portion with the correction value.

As the correction of the determination reference value, a method of correcting the determination reference value recorded in the memory circuit based on the correction value calculated from the two pieces of data described above can be mentioned.

In the present invention, the algorithm for correcting the output of the sensor portion and the algorithm for correcting the determination reference value may be performed by the arithmetic circuit of the logic portion, or may be performed by an external device.

### «Power supply portion»

In the sensor device of the present invention, the power supply portion has at least one type selected from the power generation portion and the wireless power supply portion. The power supply portion may be either one of the power generation portion and the wireless power supply portion, or may include both of these.

In the present invention, examples of the power generation portion include those formed by applying techniques, such as vibration power generation and thermoelectric power generation.

Vibration power generation is a power generation method using vibration as an energy source, and examples thereof include an electromagnetic induction method of generating power by magnetic flux change due to relative movement between a magnet and a coil, a piezoelectric method of generating electricity by applying stress to a piezoelectric element, and an electrostatic induction method of generating power by relative movement between a charged substrate and a substrate facing the substrate. The power generation portion to which the vibration power generation is applied can be formed in consideration of the description in JP2011-16061 2A.

Thermoelectric power generation is a power generation method of converting thermal energy into electric energy. The power generation portion to which the thermoelectric power generation is applied can be formed using a thermoelectric conversion element described in WO2013/069347 pamphlet or the like.

In a case where the sensor device is attached to a tooth, the vibration power generation is preferable for the power generation portion. This is because, in the oral cavity, temperature is constant and accordingly a temperature difference is difficult to obtain, but vibration is likely to occur.

As an example of the wireless power supply portion, a wireless power supply portion including a coil that can be charged in the form of magnetic energy can be mentioned. The coil is preferably formed on a semiconductor substrate using a photolithographic method. According to this aspect, since the wireless power supply portion can be formed by the semiconductor process, the productivity of the sensor device can be improved.

As a method of forming a coil using a photolithographic method, a conductor layer is formed on a semiconductor substrate or the like using a method, such as chemical vapor deposition or physical vapor deposition. Then, a photoresist is applied in the shape of a layer on the conductor layer. Then, a mask having a pattern formed thereon is overlaid on the photoresist applied in the shape of a layer, and light is emitted. Then, an uncured portion of the photoresist is removed with a developing solution to form a resist pattern. Then, using the resist pattern as a mask, the conductor layer is partially removed by etching. Then, by removing an unnecessary resist pattern, a coil can be formed.

In the present invention, it is preferable that the power supply portion further has a capacitor portion (condenser portion) for storing electric energy generated by the power generation portion or the wireless power supply portion.

### «Wireless communication portion»

In the sensor device of the present invention, the wireless communication portion transmits a determination result in the pass or fail determination, rank determination, or the like of the logic portion to the outside. As an example of the wireless communication portion, a wireless communication portion having at least an antenna circuit for transmission and an antenna circuit for reception can be mentioned.

In the present invention, from the viewpoint of power saving, it is preferable that the wireless communication portion is configured to perform wireless communication using a communication protocol enabling communication at a short distance of 0.01 m to 10 m, such as Bluetooth (registered trademark), Near Field Communication (NFC; registered trademark), Dedicated Short Range Communication (DSRC; registered trademark).

In the sensor device of the present invention, in the case of recording identification information unique to the device in the memory circuit of the logic portion, it is preferable that the wireless communication portion transmits the identification information unique to the device together with a determination result in the pass or fail determination, rank determination, or the like. According to this aspect, sensor information can be easily managed by an external device, such as a server.

In addition, by registering in advance the relationship between identification information unique to the sensor device and the position information of the sensor device, it is possible to search for sensor information around the world from big data on the server. For example, the sensor device of the present invention can be used in checking the diffusion situation of infectious diseases or the like by checking the distribution of body temperature information of human beings around the world.

In the sensor device of the present invention, it is preferable that the wireless communication portion transmits at least one piece of warning information, which is selected from the replacement timing of the sensor device and abnormalities in operation examination information, to the external device. According to this aspect, the external device that has received the warning information can transmit the warning information to the user of the sensor device by means of display or the like, and can prompt a countermeasure, such as replacing the sensor device with a new one, continuing the measurement while knowing the possibility of abnormality, or changing to a good usage with low accuracy.

### <Applications of a sensor device>

The sensor device of the present invention has low power consumption and can be miniaturized. In addition, the sensor device can be kept attached to the attachment target for a long period of time. Therefore, the sensor device of the present invention can be preferably used as a biological sensor device to be attached to a living body, such as a human being, animal, and fish.

As a method for attaching the sensor device to the living body, there is a method of implanting the sensor device in the living body or attaching the sensor device to a tooth of the living body. From the viewpoint of burden on the living body at the time of attachment and detachment, it is preferable that the sensor device is attached to a tooth.

In the case of attaching the sensor device to a tooth, for example, a method of attaching the sensor device using a dental adhesive can be mentioned.

As the dental adhesive, conventionally known dental adhesives containing polymerizable monomers or the like can be used. For example, it is possible to use an adhesive for dental implantation containing cell adhesion artificial peptides described in JP2009-7339A, a polymerizable dental composition containing a substituted sugar amide compound described in JP2013-541524A, and a dental adhesive containing a phosphate group-containing (meth) acrylate compound described in WO2013/145621 pamphlet and WO2005/060920 pamphlet.

### <Method of manufacturing a sensor device>

Next, a method of manufacturing the sensor device of the present invention will be described. The method of manufacturing itself is not part of the present invention.

The unit of the sensor device of the present invention can be manufactured by a semiconductor process.

That is, an oxide film is formed on the surface of the semiconductor substrate (step 1).

Then, a photoresist is applied in the shape of a layer on the semiconductor substrate on which the oxide film is formed (step 2).

Then, a mask having a pattern formed thereon is overlaid on the photoresist (photoresist layer) applied in the shape of a layer, and light is emitted (step 3).

Then, an uncured portion of the photoresist is removed with a developing solution to form a resist pattern (step 4).

Then, using the resist pattern as a mask, the oxide film is partially removed by etching, and then an unnecessary resist pattern is also removed (step 5). The etching may be either wet etching or dry etching.

Examples of the dry etching include ion milling, reactive ion etching, and sputter etching, and ion milling and reactive ion etching are particularly preferable. As etching gas, O₂, Cl₂, CHF₃, CF₄, SF₆, C₆₀, Ar, and the like can be mentioned.

Then, in order to make the semiconductor substrate have semiconductor characteristics, a semiconductor is formed by injecting impurity ions, such as phosphorus or boron, and performing heat treatment to activate the impurity ions (step 5).

Then, a metal layer is formed on the semiconductor (step 6).

Then, the metal layer is protected by an inorganic film for insulation (silicon oxide, silicon nitride, or the like) or an organic film (polyimide or the like), and a pattern is formed by a photolithographic process so that only a connection electrode portion in the metal layer is exposed (step 7).

In this manner, respective portions, such as a logic portion and a wireless communication portion, can be formed on the semiconductor substrate.

In the case of the sensor portion, a special semiconductor process is used in addition to or instead of the above steps, depending on the type of sensor. For example, in the case of a MEMS sensor, a method of forming irregularities or a movable portion on the semiconductor substrate by forming an inorganic film or the like after forming a sacrificial layer into a pattern using a photolithographic process and removing the sacrificial layer by dry etching is known. In the case of an image sensor, a photosensitive color resist is formed on the formed CMOS sensor circuit and a color filter is provided by a photolithographic process in many cases.

Then, the semiconductor substrates on which the respective portions are formed are bonded to each other with an adhesive, and these portions are electrically connected to each other using a technique, such as a penetrating electrode, wire bonding, and wireless electromagnetic coupling, thereby manufacturing the unit 1.

In the case of electrically connecting the respective portions to each other with a penetrating electrode, there is a method in which a via is formed in each semiconductor substrate, the inside of the via is filled with a conductive material (copper or the like), and the penetrating electrodes of the respective semiconductor substrates are bonded to each other with a solder bump or the like.

In the case of electrically connecting the respective portions to each other by wireless electromagnetic coupling, there is a method in which a coil is disposed on each semiconductor substrate so that the coils face each other between the upper and lower semiconductor substrates and the respective portions are electrically connected to each other. The coil can be formed using a photolithographic method, for example.

After forming the unit 1 as described above, at least a part of the outer periphery of the unit 1 is coated with the sealing material 2, thereby being able to manufacture the sensor device shown in Fig. 1.

As a method of coating the unit 1 with the sealing material 2, for example, a method of laminating the outer periphery of the unit 1 with a sheet-like sealing material, a method of coating the outer periphery of the unit 1 by applying a liquid sealing material to the unit 1, and the like can be mentioned. As a method of applying the liquid sealing material, a transfer molding method using a hot melted sealing material, a compression molding method, a dip coating method using a solution-like sealing material, a comma coating method, an air knife coating method, a curtain coating method, a wire bar coating method, a gravure coating method, an extrusion coating method, a spin coating method, a slit coating method, and the like can be mentioned.

### <Second example>

Next, a second example of the sensor device which is not part of the present invention will be described with reference to Fig. 7.

Fig. 7 is a schematic plan view of a unit 100.

In the unit 100 of the sensor device of the present example, a sensor portion 101, a logic portion 102, at least one type of power supply portion 103 selected from a power generation portion and a wireless power supply portion, and a wireless communication portion 104 are formed on the same semiconductor substrate 110, and these portions on the semiconductor substrate are electrically connected to each other.

The respective portions are the same as those described in the above first example.

According to the present example, a thinner sensor device can be obtained. In addition, it is possible to omit the time and effort for laminating semiconductor substrates.

The unit 100 shown in Fig. 7 can be manufactured by a semiconductor process. As described above, by performing the above-described steps 1 to 6 in a region where each portion on the semiconductor substrate is to be formed, it is possible to manufacture the unit 100 in which the sensor portion 101, the logic portion 102, at least one type of power supply portion 103 selected from a power generation portion and a wireless power supply portion, and the wireless communication portion 104 are formed on the same semiconductor substrate 110.

### <Embodiment of the invention>

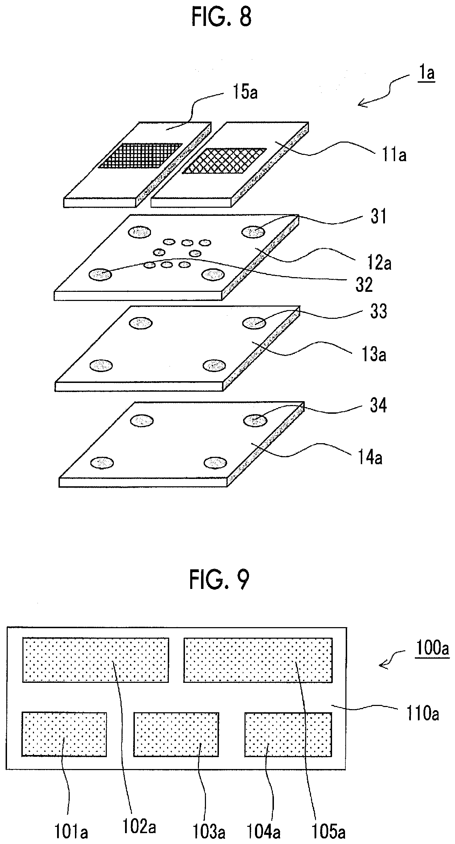

Next, an embodiment of the sensor device of the present invention will be described with reference to Fig. 8. Fig. 8 is an exploded perspective view of a unit 1a.

In the unit 1a, a semiconductor substrate 14a having a wireless communication portion, a semiconductor substrate 13a having at least one type of power supply portion selected from a power generation portion and a wireless power supply portion, and a semiconductor substrate 12a having a logic portion are laminated in this order. Then, a semiconductor substrate 11a having a sensor portion and a semiconductor substrate 15a having an energy generation portion are laminated on the semiconductor substrate 12a having a logic portion. That is, in the unit 1a, as shown in Fig. 8, the semiconductor substrate 11a having a sensor portion, the semiconductor substrate 15a having an energy generation portion, and the semiconductor substrate 14a having a wireless communication portion are disposed at the outermost layers.

It is preferable that the respective semiconductor substrates are bonded to each other with an adhesive (not shown).

Then, the respective portions formed on the semiconductor substrates that are laminated in a vertical direction are electrically connected to each other through penetrating electrodes 31 to 34. The respective penetrating electrodes are bonded to each other by solder bumps (not shown) or the like.

In the present embodiment, the sensor portion, the logic portion, the power supply portion, and the wireless communication portion are the same as those described in the above first example.

Examples of the energy generation portion include those generating at least one type of energy selected from sound, vibration, electromagnetic waves, and heat.

In this specification, the term "electromagnetic wave" means a wave formed by a change in the electric field and magnetism of the space, and includes light. For example, γ-rays, X-rays, ultraviolet light, visible light, infrared light, microwaves, radio waves, and the like can be mentioned.

In a case where an energy generation portion that generates electromagnetic waves is used, it is preferable to generate visible light, infrared light, microwaves, or radio wave in a case where a sensor device is used for a living body.

Specific examples of the energy generation portion include a light emitting diode (LED), a laser diode (LD), a semiconductor microwave generating element using a diamond substrate, a semiconductor speaker, and the like. This can be appropriately selected according to the type of the sensor portion.

For example, in a case where the sensor portion is an image sensor, a visible light sensor, or an infrared light sensor, it is preferable that the energy generation portion is an LED or an LD that generates visible to infrared light. In a case where the sensor portion is a microphone (sound sensor), it is preferable that the energy generation portion is a semiconductor speaker using a piezoelectric element or the like.

The sensor device of the embodiment is different from the sensor device of the first example in that the sensor device of the embodiment further has the semiconductor substrate 15a having an energy generation portion.

In the sensor device, since the sensor portion can perform detection using the energy output from the energy generation portion, it is possible to increase the sensor sensitivity. In addition, by shortening the energy generation pulse, the power application time of the sensor can be shortened. Therefore, it is possible to save the electric power. By periodically generating a plurality of energy generation pulses and synchronizing the detection of the sensor with this cycle, an effect that the detection S/N of the signal can be improved is also obtained.

### <Third example>

Next, a third example of the sensor device which is not part of the present invention will be described with reference to Fig. 9.

Fig. 9 is a schematic plan view of a unit 100a.

In the unit 100a of the sensor device of the present example, a sensor portion 101a, a logic portion 102a, at least one type of power supply portion 103a selected from a power generation portion and a wireless power supply portion, a wireless communication portion 104a, and an energy generation portion 105a are formed on the same semiconductor substrate 110a, and these portions on the semiconductor substrate are electrically connected to each other.

The respective portions are the same as those described in the first example and in the embodiment.

### <Fourth example>

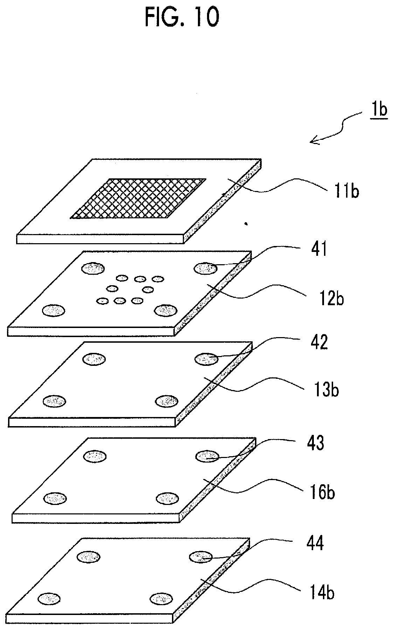

Next, a fourth example of the sensor device which is not part of the present invention will be described with reference to Fig. 10. Fig. 10 is an exploded perspective view of a unit 1b.

In the unit 1b, a semiconductor substrate 14b having a wireless communication portion, a semiconductor substrate 16b having an operation state management portion that manages the operation state of the sensor device, a semiconductor substrate 13b having at least one type of power supply portion selected from a power generation portion and a wireless power supply portion, a semiconductor substrate 12b having a logic portion, and a semiconductor substrate 11b having a sensor portion are laminated in this order. That is, in the unit 1b, as shown in Fig. 9, the semiconductor substrate 11b having a sensor portion and the semiconductor substrate 14b having a wireless communication portion are disposed at the outermost layers.

It is preferable that the respective semiconductor substrates are bonded to each other with an adhesive (not shown).

Then, the respective portions formed on the semiconductor substrates that are laminated in a vertical direction are electrically connected to each other through penetrating electrodes 41 to 44. The respective penetrating electrodes are bonded to each other by solder bumps (not shown) or the like.

In the present example, the sensor portion, the logic portion, the power supply portion, and the wireless communication portion are the same as those described in the above first example.

The operation state management portion manages the operation state of the sensor device, and it is preferable that the operation state management portion is configured to record at least one selected from the operation start date and time of the sensor device, the operating time of the sensor device, and the operation examination information of each portion of the unit and detect at least one piece of warning information selected from the replacement timing of the sensor device and an abnormality in operation examination information.

Details of the operation state management portion are similar to those described in the logic portion of the above first example.

The logic portion of the present example does not have a function as an operation state management portion.

In the unit 1b of the sensor device, the semiconductor substrate 16b having an operation state management portion, the semiconductor substrate 13b having a power supply portion, and the semiconductor substrate 12b having a logic portion are laminated in this order. However, the lamination order thereof may be changed.

The unit 1b of the sensor device may further have a semiconductor substrate having an energy generation portion.

### <Sensor system>

Next, a sensor system of the present invention will be described.

The sensor system of the present invention has the sensor device of the embodiment described above and a communication device for communication with the sensor device.

The communication device is not particularly limited as long as wireless communication with the sensor device is possible. For example, an electronic device, such as a personal computer, personal computer peripheral devices with a wireless communication function (a hard disk, a printer, and the like), home electronics with a wireless communication function (a television, a refrigerator, a telephone, and the like), and an NFC compatible device (device for reading or writing a radio frequency identification (RFID) tag or FeliCa (registered trademark)), and a mobile terminal, such as a mobile phone, a tablet, and a smartphone, can be mentioned.

Although the sensor device of the present invention may directly communicate with a server that stores big data, power consumption increases as the communication distance increases. Therefore, from the viewpoint of power saving, an external device that communicates directly with the sensor device is preferably a device that can wirelessly communicate with the sensor device using a communication protocol enabling communication at a short distance of 0.01 m to 10 m, such as Bluetooth or NFC, and that can perform long distance wireless communication (for example, Wireless Fidelity (Wi-Fi), Long Term Evolution (LTE), and the like) with the base station or the server or can be connected to the Internet (including wired connection).

In the sensor system of the present invention, the external device may be configured to perform an algorithm for determining the replacement timing of the sensor device, an algorithm for detecting an abnormality in the operation examination information of the sensor device, and an algorithm for correcting the output of the sensor portion. These algorithms can also be performed within the sensor device.

### Explanation of References

1, 1a, 1b: unit
2: sealing material
11, 11a, 11b: semiconductor substrate having a sensor portion
12, 12a, 12b: semiconductor substrate having a logic portion
13, 13a, 13b: semiconductor substrate having a power supply portion
14, 14a, 14b: semiconductor substrate having a wireless communication portion
15a: semiconductor substrate having an energy generation portion
16b: semiconductor substrate having an operation state management portion
21 to 23, 31 to 34, 41 to 44: penetrating electrode
100, 100a: unit
101, 101a: sensor portion
102, 102a: logic portion
103, 103a: power supply portion
104, 104a: wireless communication portion
105a: energy generation portion
110, 110a: semiconductor substrate

## Claims

1. A sensor device, comprising:
a unit (1a) having at least one type of power supply portion (13a) selected from a power generation portion and a wireless power supply portion, a sensor portion (11a) that is configured to detect target information and to output a signal, a logic portion (12a) that is configured to perform determination based on the signal, and a wireless communication portion (14a) that is configured to transmit a result of the determination to an outside; and
a sealing material (2) that covers at least a part of an outer periphery of the unit (1a),
wherein the respective portions are formed on a plurality of semiconductor substrates, the semiconductor substrates on which the respective portions are formed are laminated, and the respective portions are electrically connected to each other;
wherein the wireless communication portion (14a), the power supply portion (13a), the logic portion (12a) and the sensor portion (11a) are stacked in this order in a vertical direction such that the portions overlap each other,
wherein the semiconductor substrate having the sensor portion (11a) and a semiconductor substrate having an energy generation portion (15a) are stacked on the semiconductor substrate having a logic portion (12a); the energy generation portion being configured to generate at least one type of energy selected from sound, vibration, electromagnetic waves, and heat; and
wherein the sensor portion (11a) is configured to perform detection using energy output from the energy generation portion (15a) and the energy generation portion (15a) is configured to generate a plurality of energy generation pulses with which detection of the sensor portion is synchronized.

2. The sensor device according to claim 1, wherein the unit (1a) has a shape selected from the group consisting a rectangular shape, a sheet shape, a spherical shape, and an ellipsoid shape.

3. The sensor device according to claim 1 or 2,
wherein the sealing material (2) covers the entire outer periphery of the unit (1a).

4. The sensor device according to any one of claims 1 to 3,
wherein the unit (1a) has an analog-to-digital conversion portion that is configured to digitally convert the signal output from the sensor portion (11a).

5. The sensor device according to any one of claims 1 to 4,
wherein the wireless communication portion (14a) is configured to transmit identification information unique to the sensor device to the outside.

6. The sensor device according to any one of claims 1 to 5,
wherein the sensor device is for a living body.

7. The sensor device according to any one of claims 1 to 6,
wherein the sensor device is attached to a tooth.

8. The sensor device according to claim 1, wherein the lamination of the wireless communication portion (14a), the power supply portion (13a), the logic portion (12a), and the sensor portion (11a) are electrically connected to each other through penetrating electrodes.

9. The sensor device according to claim 1, wherein the wireless communication portion (14a), the power supply portion (13a), the logic portion (12a), and the sensor portion (11a) are in contact with each other on the surfaces thereof.

10. A sensor system, comprising:
the sensor device according to any one of claims 1 to 9; and
a communication device that is configured to communicate with the sensor device.

## Patentansprüche

1. Sensorvorrichtung, umfassend:
eine Einheit (1a), die mindestens eine Art von Energieversorgungsabschnitt (13a), der aus einem Energieerzeugungsabschnitt und einem drahtlosen Energieversorgungsabschnitt ausgewählt ist, einen Sensorabschnitt (11a), der konfiguriert ist, um Zielinformationen zu erkennen und ein Signal auszugeben, einen Logikabschnitt (12a), der konfiguriert ist, um eine Bestimmung basierend auf dem Signal durchzuführen, und einen drahtlosen Kommunikationsabschnitt (14a) aufweist, der konfiguriert ist, um ein Ergebnis der Bestimmung nach außen zu senden; und
ein Dichtungsmaterial (2), das zumindest einen Teil des Außenumfangs der Einheit (1a) abdeckt,
wobei die jeweiligen Abschnitte auf einer Vielzahl von Halbleitersubstraten ausgebildet sind, die Halbleitersubstrate, auf denen die jeweiligen Abschnitte ausgebildet sind, laminiert sind, und die jeweiligen Abschnitte elektrisch miteinander verbunden sind;
wobei der drahtlose Kommunikationsabschnitt (14a), der Energieversorgungsabschnitt (13a), der Logikabschnitt (12a) und der Sensorabschnitt (11a) in dieser Reihenfolge in einer vertikalen Richtung gestapelt sind, so dass die Abschnitte einander überlappen,
wobei das Halbleitersubstrat mit dem Sensorabschnitt (11a) und ein Halbleitersubstrat mit einem Energieerzeugungsabschnitt (15a) auf dem Halbleitersubstrat mit einem Logikabschnitt (12a) gestapelt sind; wobei der Energieerzeugungsabschnitt konfiguriert ist, um mindestens eine Art von Energie zu erzeugen, die aus Schall, Vibration, elektromagnetischen Wellen und Wärme ausgewählt ist; und
wobei der Sensorabschnitt (11a) konfiguriert ist, um eine Erkennung unter Verwendung von Energie durchzuführen, die von dem Energieerzeugungsabschnitt (15a) ausgegeben wird, und der Energieerzeugungsabschnitt (15a) konfiguriert ist, um eine Vielzahl von Energieerzeugungsimpulsen zu erzeugen, mit denen die Erkennung des Sensorabschnitts synchronisiert wird.

2. Sensorvorrichtung nach Anspruch 1, wobei die Einheit (1a) eine Form aufweist, die aus der Gruppe ausgewählt ist, die aus einer rechteckigen Form, einer Blattform, einer kugelförmigen Form und einer ellipsoiden Form besteht.

3. Sensorvorrichtung nach Anspruch 1 oder 2,
wobei das Dichtungsmaterial (2) den gesamten Außenumfang der Einheit (1a) abdeckt.

4. Sensorvorrichtung nach einem der Ansprüche 1 bis 3,
wobei die Einheit (1a) einen Analog-Digital-Wandlerabschnitt aufweist, der konfiguriert ist, um das von dem Sensorabschnitt (11a) ausgegebene Signal digital zu wandeln.

5. Sensorvorrichtung nach einem der Ansprüche 1 bis 4,
wobei der drahtlose Kommunikationsabschnitt (14a) konfiguriert ist, um Identifikationsinformationen, die für die Sensorvorrichtung einzigartig sind, nach außen zu senden.

6. Sensorvorrichtung nach einem der Ansprüche 1 bis 5,
wobei die Sensorvorrichtung für einen lebenden Körper bestimmt ist.

7. Sensorvorrichtung nach einem der Ansprüche 1 bis 6,
wobei die Sensorvorrichtung an einem Zahn angebracht ist.

8. Sensorvorrichtung nach Anspruch 1, wobei die Laminierung des drahtlosen Kommunikationsabschnitts (14a), des Energieversorgungsabschnitts (13a), des Logikabschnitts (12a) und des Sensorabschnitts (11a) durch durchdringende Elektroden elektrisch miteinander verbunden sind.

9. Sensorvorrichtung nach Anspruch 1, wobei der drahtlose Kommunikationsabschnitt (14a), der Energieversorgungsabschnitt (13a), der Logikabschnitt (12a) und der Sensorabschnitt (11a) an ihren Oberflächen miteinander in Kontakt sind.

10. Sensorsystem, umfassend:
die Sensorvorrichtung nach einem der Ansprüche 1 bis 9; und
eine Kommunikationsvorrichtung, die konfiguriert ist, um mit der Sensorvorrichtung zu kommunizieren.

## Revendications

1. Dispositif capteur, comprenant :
une unité (1a) présentant au moins un type de portion d'alimentation en électricité (13a) sélectionné parmi une portion de génération d'électricité et une portion d'alimentation en électricité sans fil, une portion capteur (11a) qui est configurée pour détecter des informations cibles et pour émettre un signal, une portion logique (12a) qui est configurée pour réaliser une détermination sur la base du signal, et une portion de communication sans fil (14a) qui est configurée pour transmettre un résultat de la détermination à un extérieur ; et
un matériau de scellement (2) qui couvre au moins une partie d'une périphérie extérieure de l'unité (1a),
dans lequel les portions respectives sont formées sur une pluralité de substrats semi-conducteurs, les substrats semi-conducteurs sur lesquels les portions respectives sont formées sont stratifiés, et les portions respectives sont connectées électriquement les unes aux autres ;
dans lequel la portion de communication sans fil (14a), la portion d'alimentation en électricité (13a), la portion logique (12a) et la portion capteur (11a) sont empilées dans cet ordre dans une direction verticale de sorte que les portions se chevauchent les unes les autres,
dans lequel le substrat semi-conducteur présentant la portion capteur (11a) et un substrat semi-conducteur présentant une portion de génération d'énergie (15a) sont empilés sur le substrat semi-conducteur présentant une portion logique (12a) ; la portion de génération d'énergie étant configurée pour générer au moins un type d'énergie sélectionné parmi un son, une vibration, des ondes électromagnétiques et de la chaleur ; et
dans lequel la portion capteur (11a) est configurée pour réaliser une détection en utilisant de l'énergie émise à partir de la portion de génération d'énergie (15a) et la portion de génération d'énergie (15a) est configurée pour générer une pluralité d'impulsions de génération d'énergie sur lesquelles la détection de la portion capteur est synchronisée.

2. Dispositif capteur selon la revendication 1, dans lequel l'unité (1a) présente une forme sélectionnée parmi le groupe consistant en une forme rectangulaire, une forme de feuille, une forme sphérique et une forme ellipsoïdale.

3. Dispositif capteur selon la revendication 1 ou 2,
dans lequel le matériau de scellement (2) couvre la périphérie extérieure entière de l'unité (1a).

4. Dispositif capteur selon l'une quelconque des revendications 1 à 3,
dans lequel l'unité (1a) présente une portion de conversion analogique-numérique qui est configurée pour convertir numériquement le signal émis à partir de la portion capteur (11a).

5. Dispositif capteur selon l'une quelconque des revendications 1 à 4,
dans lequel la portion de communication sans fil (14a) est configurée pour transmettre des informations d'identification uniques au dispositif capteur à l'extérieur.

6. Dispositif capteur selon l'une quelconque des revendications 1 à 5,
dans lequel le dispositif capteur est destiné à un corps vivant.

7. Dispositif capteur selon l'une quelconque des revendications 1 à 6,
dans lequel le dispositif capteur est attaché à une dent.

8. Dispositif capteur selon la revendication 1, dans lequel la stratification de la portion de communication sans fil (14a), la portion d'alimentation en électricité (13a), la portion logique (12a) et la portion capteur (11a) sont connectées électriquement les unes aux autres par le biais d'électrodes pénétrantes.

9. Dispositif capteur selon la revendication 1, dans lequel la portion de communication sans fil (14a), la portion d'alimentation en électricité (13a), la portion logique (12a) et la portion capteur (11a) sont en contact les unes avec les autres sur les surfaces de celles-ci.

10. Système capteur, comprenant :
le dispositif capteur selon l'une quelconque des revendications 1 à 9 ; et
un dispositif de communication qui est configuré pour communiquer avec le dispositif capteur.
